# EUROPEAN PATENT APPLICATION

(11) **EP 4 591 904 A1**
(43) Date of publication of application: **30.07.2025**
(21) Application number: 25153364.2
(22) Date of filing: 22.01.2025
(51) Int. Cl.: A61M 5/178, A61J 1/20

(54) **FLUID RESERVOIRS AND ASSOCIATED DEVICES AND METHODS**

(30) Priority: 26.01.2024 US 202463625757 P; 01.10.2024 US 202463702074 P; 13.01.2025 US 202519018041
(71) Applicant: Medtronic MiniMed, Inc., Northridge, CA 91325 (US)
(72) Inventor: Brockman, Rachel K., Northridge, 91325 (US); Larson, Eric A., Northridge, 91325 (US)
(74) Representative: Zimmermann & Partner Patentanwälte mbB

(57) **Abstract**

Fluid reservoir systems are disclosed herein. According to some embodiments, the present technology includes a reservoir defining a cavity and a plunger slidably received within the cavity. The plunger includes a vial interface, a plunger rod extending distally from the vial interface, and a needle extending longitudinally through the plunger rod. The needle has an inlet end at the vial interface and an outlet end proximate a distal portion of the plunger rod. When an outlet end of a vial of fluid medication is coupled to the vial interface, the needle extends through a septum of the vial such that the inlet end of the needle is disposed within the vial. When the plunger is fluidly coupled to the vial, proximal movement of the plunger and vial relative to the reservoir draws the fluid medication out of the vial, through the needle, and into the interior region of the reservoir.

## Description

### CROSS-REFERENCE TO RELATED APPLICATION(S)

The present application claims the benefit of priority to U.S. Provisional Application No. 63/625,757, filed January 26, 2024, and U.S. Provisional Application No. 63/702,074, filed October 1, 2024, both of which are incorporated by reference herein in their entireties.

### TECHNICAL FIELD

The present technology relates to fluid delivery systems. In particular, the present technology relates to fluid reservoirs and associated devices and methods for filling fluid reservoirs with a fluid medication.

### BACKGROUND

Certain diseases or conditions may be treated, according to modern medical techniques, by delivering a medication fluid or other substance to the body of a patient, either in a continuous manner or at particular times or time intervals within an overall time period. For example, diabetes is commonly treated by delivering defined amounts of insulin to the patient at appropriate times. Some common modes of providing insulin therapy to a patient include delivery of insulin through manually operated syringes and insulin pens. Other modern systems employ programmable fluid infusion devices (e.g., continuous insulin infusion devices such as insulin pumps) to deliver controlled amounts of insulin or other drugs to a patient.

Persistent problems associated with systems designed to infuse medication include having components that can be difficult for some patients to use, and also that the human body spontaneously reacts against foreign bodies which are introduced into the body, such as an implanted cannula. Among the various responses of a body to foreign bodies, inflammation and the build-up of fibrous tissue at the infusion site significantly shortens the duration that an infusion set may be maintained at a single infusion site (i.e. "site-loss"). Moreover, tissue encapsulation and blockage of the implanted cannula or catheter (i.e. "occlusion") often occurs, thereby impeding or halting infusion of medication. Inflammation may be accelerated due to agglomerations that develop in the insulin as the insulin is stored in a reservoir at ambient temperature within the pump. Thus, frequent re-positioning of the infusion site for continued usage of the infusion pump is required. Currently, wear times of all the commercial insulin infusion sets are labeled for < 3 days.

Infusion systems often include a fluid infusion device (e.g., a pumping device) and an infusion set including a connector to attach the infusion set to the pumping device, a cannula or a needle that is implanted into a user, and a tubing section between the connector and the cannula or needle to carry medication from a reservoir in the pumping device to the user. There are many types of infusion sets that may be connected to a single pumping device, each often selected by a user based on personal preferences. Other difficulties with existing infusion systems stem from the user having to fill the pump reservoir with insulin provided in a separate vial. The process of transferring insulin from the vial to the reservoir can be both difficult and error prone. Challenging aspects include needing to maintain sterility of multiple components and contacting surfaces, the logistics of using an intermediate transfer device (such as a syringe), mismeasurement of the insulin, and the inadvertent introduction of air into the reservoir. While these difficulties can be inconvenient, the resulting errors can result in more serious problems such as incorrect insulin dosing, which can lead to acute hypoglycemia or chronic hyperglycemia.

Accordingly, there is a need for improved fluid reservoirs and associated devices and methods for filling fluid reservoirs.

### SUMMARY

The present technology includes a fluid transfer assembly comprising a reservoir and a plunger. The reservoir comprises a sidewall having a proximal end and a distal end and that surrounds and defines an interior cavity. The sidewall has an opening at the proximal end. The reservoir is configured to receive and contain a fluid medication within the interior cavity. The plunger has a proximal end portion and a distal end portion. The plunger comprises a vial interface at the proximal end portion configured to receive an outlet end of a vial containing a fluid medication, a plunger rod extending distally from the vial interface and slidably disposed within the interior cavity of the reservoir, and a needle extending longitudinally through the plunger rod. The needle has an inlet end at the vial interface and an outlet end proximate a distal portion of the plunger rod. The needle defines a lumen extending therethrough. The fluid transfer assembly is configured to be coupled to a vial such that, when the outlet end of the vial is coupled to the vial interface of the plunger, the needle extends through a septum of the vial such that the inlet end of the needle is disposed within the vial and in fluid communication with the fluid medication. When the needle is fluidly coupled to the vial, proximal movement of the plunger and vial relative to the reservoir draws the fluid medication out of the vial, through the needle, and into the interior region of the reservoir.

In some embodiments, the vial interface of the plunger comprises an annular sidewall defining a cavity therein, and the inlet end of the needle is disposed within the cavity, distal of the proximal end of the annular sidewall. In several of such embodiments, the annular sidewall comprises a flange extending around at least a portion of the circumference of an inner surface of the annular sidewall. The flange projects into the cavity and is configured to engage the outlet end of the vial to secure the outlet end within the cavity.

According to several embodiments, the plunger includes a plunger head coupled to the distal portion of the plunger rod, and the plunger head extends between an outer surface of the distal portion of the plunger rod and an inner surface of the sidewall of the reservoir, thereby creating a seal between the plunger and the reservoir. The plunger head may comprise a septum, and the needle extends through the septum such that the outlet end of the needle is distal of a distal end of the septum. In several embodiments, the plunger head extends circumferentially around the distal portion of the plunger rod and includes an inner surface defining a plurality of threads configured to engage a plurality of threads at the outer surface of the distal portion of the plunger rod. In some examples, the reservoir is configured to be received within a fluid infusion pump. According to some embodiments, wherein a distal portion of the reservoir includes a filter configured to filter out particulates from the fluid medication when the fluid medication is dispensed from the fluid reservoir.

The present technology includes a method for transferring a fluid medication from a vial to a reservoir. The method comprises providing a fluid transfer assembly that includes a reservoir defining an interior cavity configured to receive and contain a fluid medication and a plunger. The plunger has a proximal end portion and a distal end portion. The plunger comprises a vial interface at the proximal end portion, a plunger rod extending distally from the vial interface, and a needle. The plunger rod is slidably positioned within the interior cavity of the reservoir. The needle extends longitudinally through the plunger and has an inlet end at the vial interface and an outlet end proximate a distal portion of the plunger rod. The needle defines a lumen extending therethrough. The method further comprises coupling an outlet end of a vial containing a fluid medication to the vial interface of the plunger thus piercing a septum of the vial with the inlet end of the needle and establishing a fluid connection between the vial and the lumen of the needle. While the vial remains engaged to the vial interface of the plunger, the method includes moving the vial and plunger together in a proximal direction, away from the reservoir, thereby creating a negative pressure within the interior region of the reservoir and drawing the fluid medication out of the vial, through the needle, and into the interior region of the reservoir. In some embodiments, coupling the outlet end of the vial to the vial interface comprises inserting the outlet end of the vial distally into a cavity at the proximal end of the plunger.

The method may further comprise detaching the vial from the plunger after drawing a desired amount of fluid medication into the interior region of the reservoir. In some cases, the method further comprises removing the plunger from the reservoir after drawing a desired amount of fluid medication into the interior region of the reservoir. The plunger may further comprise a plunger head coupled to the distal portion of the plunger rod and extending between an outer surface of the plunger rod and an inner surface of the reservoir. In such embodiments, the method may further include detaching the plunger rod from the plunger head to enable removal of the plunger from the reservoir, and removing the plunger from the reservoir while the plunger head remains within the reservoir and preventing egress of the fluid medication. In some cases, detaching the plunger rod from the plunger head comprises rotating the plunger rod relative to the plunger head. Any of the foregoing methods may include placing the reservoir in a fluid infusion pump.

Several embodiments of the present technology include a reservoir for use with a fluid infusion system for delivering a fluid medication to a user. The reservoir may comprise a barrel portion defining an interior cavity configured to receive a fluid medication and a dispensing portion including a septum. The dispensing portion extends distally from the barrel portion and defines a channel therein. The channel has a proximal end fluidly coupled to the barrel portion and terminates distally at the septum. When the fluid medication is dispensed from the reservoir, the fluid medication flows along a flow path starting in the barrel portion and extending through the channel and into a needle extending through the septum. The reservoir further includes a filter disposed in the flow path within the channel of the dispensing portion, the filter configured to trap particulates in the fluid medication.

In some embodiments of the reservoir, the dispensing portion comprises a neck extending distally from the barrel portion and a head extending distally away from the neck. The head has a greater cross-sectional dimension than a cross-sectional dimension of the neck, and at least a portion of the filter is positioned within a portion of the channel extending through the neck. In some examples, the septum is disposed within the head of the dispensing region, the head further includes a receiving cavity comprising a portion of the channel between a distal end of the filter and a proximal end of the septum, and the receiving cavity is configured to receive an inlet end of a needle extending proximally through the septum. According to some embodiments, the reservoir can further comprise a cap configured to be positioned over the dispensing region to secure the septum within the dispensing region. In particular embodiments, the filter is a first filter and the reservoir further comprises a second filter disposed within the dispensing region in the flow path. In certain examples, the dispensing portion comprises a neck extending distally from the barrel portion and a head extending distally away from the neck, and the head has a greater cross-sectional dimension than a cross-sectional dimension of the neck. The filter is a first filter and is positioned within a portion of the channel extending through the neck, and the dispensing portion further includes a second filter disposed within the channel, downstream of the first filter.

Further disclosed herein are fluid reservoir systems. According to some embodiments, the present technology includes a reservoir defining a cavity and a plunger slidably received within the cavity. The plunger includes a vial interface, a plunger rod extending distally from the vial interface, and a needle extending longitudinally through the plunger rod. The needle has an inlet end at the vial interface and an outlet end proximate a distal portion of the plunger rod. When an outlet end of a vial of fluid medication is coupled to the vial interface, the needle extends through a septum of the vial such that the inlet end of the needle is disposed within the vial. When the plunger is fluidly coupled to the vial, proximal movement of the plunger and vial relative to the reservoir draws the fluid medication out of the vial, through the needle, and into the interior region of the reservoir.

### BRIEF DESCRIPTION OF THE DRAWINGS

Many aspects of the present disclosure can be better understood with reference to the following drawings. The components in the drawings are not necessarily to scale. Instead, emphasis is placed on illustrating clearly the principles of the present disclosure.
FIGS. 1A and 1B are front and cross-sectional views, respectively, of a fluid transfer assembly configured in accordance with several embodiments of the present technology.
FIG. 2A is an enlarged cross-sectional view of a distal portion of the fluid transfer assembly shown in FIGS. 1A and 1B.
FIG. 2B is an enlarged cross-sectional view of a distal portion of another embodiment of a fluid transfer assembly configured in accordance with the present technology.
FIG. 3 is an isolated view of a plunger rod of the fluid transfer assembly shown in FIGS. 1A, 1B, and 2.
FIGS. 4A-4D show a method of filling a fluid reservoir with the fluid transfer assembly of FIGS. 1A-3 in accordance with several embodiments of the present technology.
FIGS. 5A and 5B show the distal portion of a fluid transfer assembly with the plunger in a partially advanced and fully advanced position, in accordance with several embodiments of the present technology.
FIGS. 6A, 6B, and 6C show various plunger heads for use with the fluid transfer assemblies of the present technology.
FIG. 7 shows a fluid transfer assembly of the present technology coupled to a vial and including a grip component, in accordance with several embodiments of the present technology.
FIG. 8 shows an infusion pump coupled to an infusion set, with the reservoir of the present technology received within the infusion pump.

### DETAILED DESCRIPTION

The present technology relates to certain components and features of fluid infusion systems for treating a medical condition of a patient. In particular, the present technology relates to fluid reservoirs and associated devices and methods for filling fluid reservoirs with a fluid medication. The non-limiting examples described below relate to a medical device used to treat diabetes (more specifically, an insulin pump), although embodiments of the disclosed subject matter are not so limited. Accordingly, the fluid medication is insulin in certain embodiments. In other embodiments, however, fluid medications other than insulin may be administered through the described fluid infusion systems such as, for example, drugs to treat pulmonary hypertension, iron chelation drugs, pain medications, anti-cancer treatments, medications, vitamins, hormones, or the like.

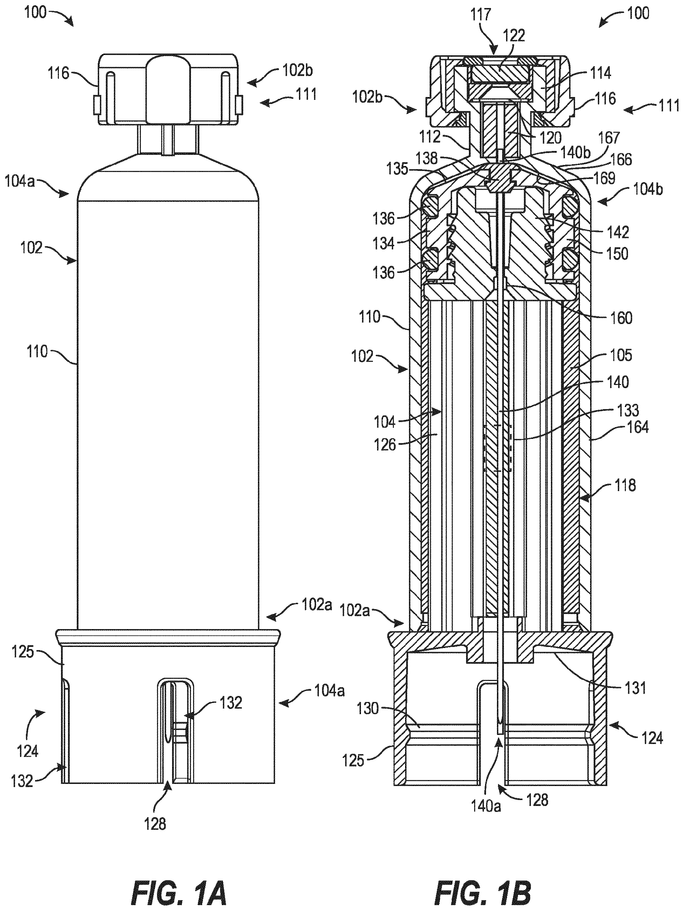

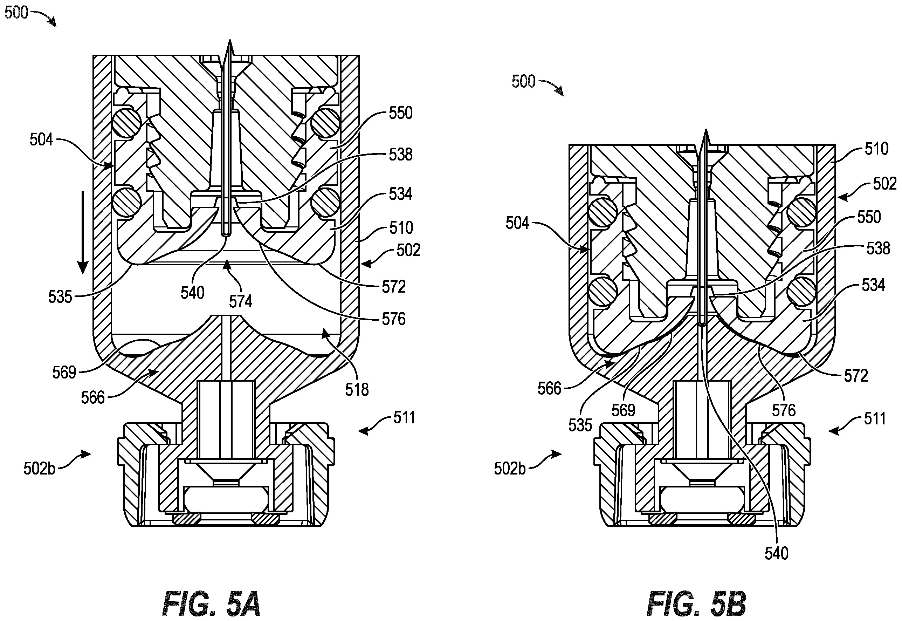

FIGS. 1A and 1B are front and cross-sectional views, respectively, of a fluid transfer assembly 100 (or "transfer assembly 100") configured in accordance with the present technology. The fluid transfer assembly 100 is configured to facilitate transfer of a fluid medication from a vial (not shown) to a fluid reservoir 102 prior to coupling the reservoir 102 to a fluid infusion device for delivery of the fluid medication to a patient. As shown in FIGS. 1A and 1B, the fluid transfer assembly 100 includes the reservoir 102 and a plunger 104. The plunger 104 comprises a plunger rod 105 and a plunger head 150 disposed at a distal end of the plunger rod 105. Prior to a reservoir filling procedure, the plunger 104 is disposed within the barrel of the reservoir 102 (as depicted). At the completion of the procedure, the plunger rod 105 is removed from the plunger head 150 of the plunger 104 and the reservoir 102 can be operatively coupled to an infusion pump or other fluid delivery system (as shown, for example, in FIG. 5 below).

The reservoir 102 extends longitudinally between a proximal end region 102a and a distal end region 102b, and the plunger 104 extends longitudinally between a proximal end portion 104a and a distal end portion 104b. As used herein, "proximal" refers to a position along the longitudinal axis of the transfer assembly 100 that is closer to the open end of the plunger rod 105 (into which a fluid medication vial is received), and "distal" refers to a position along the longitudinal axis of the transfer assembly 100 that is farther from the open end of the plunger rod 105. When the reservoir 102 is later coupled to a fluid delivery system for dispensation of the fluid medication, the fluid medication flows in a proximal to distal direction.

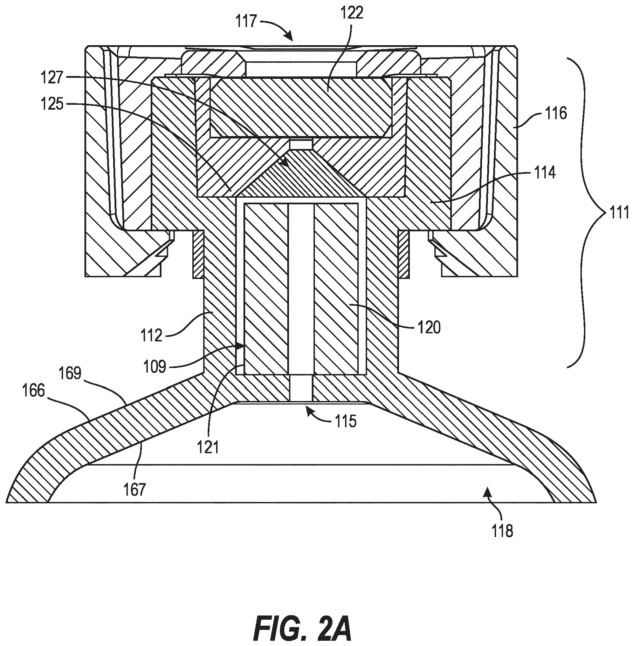

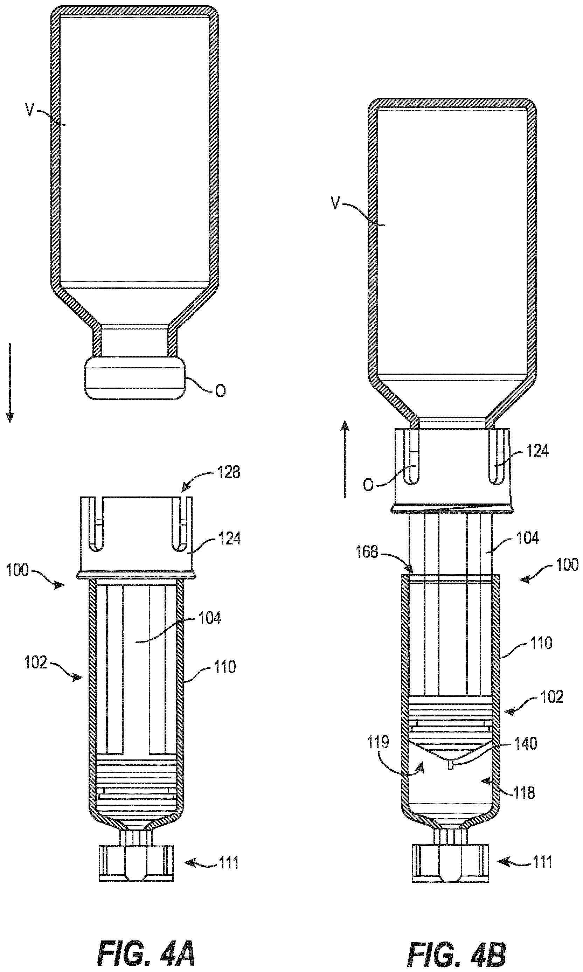

FIG. 2A is an enlarged view of the distal end region 102b of the reservoir 102. With reference to FIGS. 1A-2A, the fluid reservoir 102 can comprise a barrel portion 110 and a dispensing portion 111 extending distally from the barrel portion 110. The barrel portion 110 defines an interior region 118 configured to receive and store the fluid medication, as well as slidably receive the plunger 104 therein. The interior region 118 is bound by a sidewall having a body portion 164 and an end portion 166. The body portion 164 has an opening 168 (best visualized in FIG. 4B) at its proximal end, and the end portion 166 extends distally from a distal end of the body portion 164. The plunger 104 is configured to be received through the opening 168 during assembly. The cross-sectional dimension of the outer surface 167 of the sidewall along the end portion 166 may taper distally. The inner surface 169 of the sidewall along the end portion 166 may also taper distally (as shown in FIGS. 1A-2A), generally following the contour of the outer surface 167 and/or the distal face of the plunger 104. At least by mirroring the distal face of the plunger 104, dead space within the reservoir 102 once the plunger 104 is advanced fully within the barrel portion 110 (as shown in FIGS. 1A and 1B) is reduced or eliminated. In some embodiments, the contour of the inner surface 169 of the sidewall along the end portion 166 may not follow the contour of the outer surface 167, for example as discussed below with reference to FIGS. 5A-6C.

The dispensing portion 111 comprises a narrowed portion of the reservoir 102 and defines a channel 109 (see FIG. 2A) through which the fluid medication travels when being dispensed from the barrel portion 110 into an infusion set and ultimately into a user. Alternatively, in a patch-style infusion device, the fluid medication may travel from a reservoir 102 into a cannula that is directly inserted into a user. The interior region 118 of the barrel portion 110 and the channel 109 of the dispensing portion 111 are fluidly coupled via a passage 115 (see FIG. 2A) extending therebetween. The dispensing portion 111 can further include a septum 122 at a distal end of the channel 109. As such, the channel 109 can extend from a distal end of the passage 115 to the septum 122.

The dispensing portion 111 can include a neck region 112 that extends distally from the barrel portion 110 and a head region 114 that extends distally from the neck region 112. As shown, the head region 114 may have a cross-sectional dimension greater than a cross-sectional dimension of the neck region 112. In some embodiments, the distal end region 102b of the reservoir 102 further includes a cap 116 coupled to the head region 114 to help secure the septum 122 to the head region 114. The cap 116 may have an opening 117 overlying a portion of the septum 122 and through which a needle can be received for piercing the septum 122.

The reservoir 102 further includes a filter 120 disposed within the channel 109 of the dispensing portion 111 and configured to filter out particulates from the fluid medication (such as insulin aggregates) as the fluid medication passes through the channel 109. Filtering of particulates from the fluid medication reduces the potential for inflammation at the infusion site on the patient and advantageously prolongs the duration of cannula implantation (for example, up to 7 days or more). The filter 120 may comprise a single filter (as shown) or a plurality of filters (e.g., two filter, three filters, four filters, etc.). The filter 120 can comprise a porous, hydrophilic structure formed of polyvinyl alcohol (PVA), polyurethane, polyester, polyether, polyacrylate, nylon, cellulose, cellulose acetate, cellulose nitrate, polyethylene, polyvinyl acetate, polysulfone, polyethersulfone (PES), collagen, polycaprolactone, an acrylic copolymer, mixed cellulose esters, polytetrafluoroethylene (PTFE), polycarbonate, and other hydrophilic materials. In some embodiments, one, some, or all of the filters can include a hydrophilic additive, such as glycerol. Additional details regarding the filter assembly can be found in U.S. Provisional Application No. 63/625,721, titled FLUID DELIVERY FILTERS, filed January 26, 2024, U.S. Patent No. 11,197,949, titled MEDICATION INFUSION COMPONENTS AND SYSTEMS, filed January 18, 2018, and U.S. Patent No. 10,279,126, titled FLUID CONDUIT ASSEMBLY WITH GAS TRAPPING FILTER IN THE FLUID FLOW PATH, filed October 7, 2014, all of which are incorporated by reference herein in their entireties.

In some embodiments, the reservoir 102 includes a first filter (such as filter 120 shown in FIG. 2A) and a second filter (not shown) within the channel 109 downstream of the first filter. The first filter may comprise an expandable porous structure with a first average pore size and a first height, and the second filter can comprise a second porous structure with a second average pore size and a second height. The second average pore size can be less than the first average pore size, and the second height can be less than the first height. The second filter, for example, can be configured to trap smaller aggregates that pass through the larger pores of the first filter. In some embodiments, the first filter may comprise PVA, a cellulose, a polyurethane, a polyester, a polyether, or a collagen, and may have an average pore size of from about 0.1 mm to about 0.5 mm, while the second filter may comprise an acrylic copolymer membrane, a polyethersulfone membrane, a mixed cellulose esters membrane, a cellulose acetate membrane, a cellulose nitrate membrane, a nylon membrane, a hydrophilic polytetrafluoroethylene (PTFE) membrane, and/or a polycarbonate membrane, and may have an average pore size of from about 0.1 µm to about 10 µm.

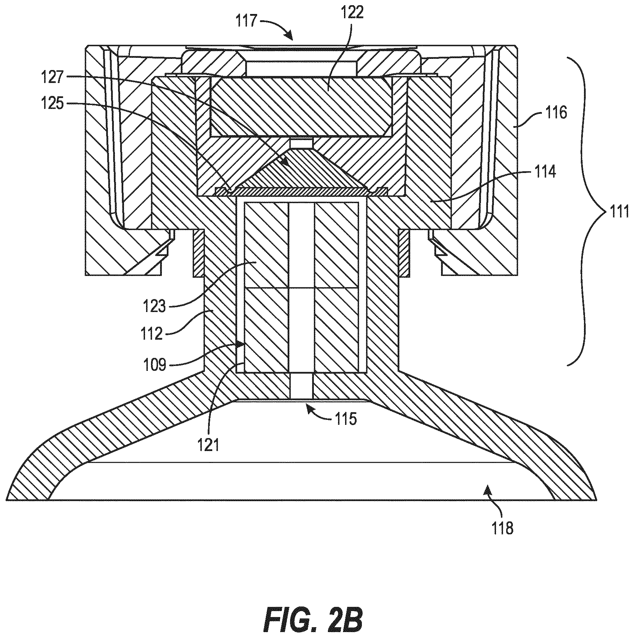

FIG. 2B is an enlarged cross-sectional view of a distal portion of another embodiment of a reservoir configured in accordance with the present technology. The reservoir represented by the distal portion in FIG. 2B can have the same components and features as the reservoir of FIGS. 1A-2A, except the reservoir of FIG. 2B includes a plurality of filters. Each of the filters can comprise a porous, hydrophilic structure, formed of polyvinyl alcohol (PVA), polyurethane, polyester, polyether, polyacrylate, nylon, cellulose, cellulose acetate, cellulose nitrate, polyethylene, polyvinyl acetate, polysulfone, polyethersulfone (PES), collagen, polycaprolactone, an acrylic copolymer, mixed cellulose esters, polytetrafluoroethylene (PTFE), polycarbonate, and other hydrophilic materials. In some embodiments, one, some, or all of the filters can include a hydrophilic additive, such as glycerol,

In the embodiment depicted in FIG. 2B, the reservoir 102 includes three filters: a first filter 121 disposed within the channel 109 proximate the passage 115; a second filter 123 disposed within the channel 109 distal of the first filter 121; and a third filter 125 disposed within the channel 109 distal of the second filter 123. Each of the filters can generally span a width of the channel 109 such that the fluid medication is forced to travel through the filters when being dispensed. The first, second, and third filters 121, 123, and 125 can be formed of the same or different materials, have the same or different pore sizes, have the same or different porosities, have the same or different widths, and have the same or different heights. In other examples, the filter assembly 120 can comprise a single filter, two filters, four filters, etc. Additional details regarding the filter assembly can be found in U.S. Provisional Application No. 63/625,721, titled FLUID DELIVERY FILTERS, filed January 26, 2024, U.S. Patent No. 11,197,949, titled MEDICATION INFUSION COMPONENTS AND SYSTEMS, filed January 18, 2018, and U.S. Patent No. 10,279,126, titled FLUID CONDUIT ASSEMBLY WITH GAS TRAPPING FILTER IN THE FLUID FLOW PATH, filed October 7, 2014, all of which are incorporated by reference herein in their entireties.

The dispensing portion 111 can further include a receiving zone 127 comprising a portion of the channel 109 between a distal end of the filter 120 and a proximal end of the septum 122. When a needle (e.g., of an infusion set cap or other fluid conduit component) is positioned across the septum 122 during a fluid dispensing operation and receiving fluid from the channel 109, a piercing/inlet end of the needle can be positioned within the receiving zone 127, distal of the filter 120, to avoid piercing or otherwise contacting any portion of the filter 120 with the needle. The dispensing portion 111 can be configured to ensure the filter 120 remains proximal of the distal tip of the needle when the needle is inserted so that the dispensed fluid medication passes through (and is filtered by) the filter 120 before flowing through the needle. This way, the needle does not clog with insulin aggregates or other particulates.

Referring again to FIGS. 1A and 1B, the plunger 104 comprises a plunger rod 105 and a plunger head 150 disposed at the distal end portion of the plunger rod 105. The plunger rod 105 may comprise a vial interface 124 coinciding with the proximal end portion 104a of the plunger 104, a shaft 126 extending distally from the vial interface 124, and a plunger head coupling region 142 coinciding with the distal end portion 104b of the plunger 104. The shaft 126 is configured to be slidably disposed within the interior region 118 of the barrel portion 110 of the reservoir 102 during a fluid transfer operation while the vial interface 124 remains proximal and external of the barrel portion 110.

The vial interface 124 can be configured to receive and secure an outlet end of a fluid medication vial (shown below in detail in FIGS. 4A-4D) during a fluid transfer procedure. The vial interface 124 may comprise an annular sidewall 129 extending proximally from a base 131 (FIG. 1B) of the shaft 126, and together the annular sidewall 129 and base 131 define a cavity 128 at the proximal end portion 104a of the plunger 104. In some embodiments, the vial interface 124 can include a means for releasably securing the outlet end of the vial within the cavity 128. For example, as shown in FIG. 1B, in some examples the annular sidewall 129 includes a flange 130 extending around at least a portion of the circumference of an inner surface of the annular sidewall 129. The flange 130 can project inwardly into the cavity 128 and, when the outlet end of the vial has been inserted distally beyond the flange 130, the flange 130 resists proximal movement of the vial, thereby securing the vial within the cavity 128. In these and other embodiments, the annular sidewall 129 can optionally include one or more windows 132 that enable a user to view the position of the needle and the outlet end of the vial during and after insertion of the vial and enable the annular sidewall to flex outwardly when a vial is inserted into the cavity 128.

The plunger 104 further includes a plunger head 150 coupled to the plunger head coupling region 142 at the distal portion of the shaft 126. The plunger head 150 extends between an outer surface of the plunger 104 and an inner surface of the sidewall of the reservoir 102, thereby creating a fluid seal between the plunger 104 and the reservoir 102. In some embodiments, the plunger head 150 comprises a plunger head body 134 extending around a circumference of the plunger head coupling region 142 and over the distal face of the plunger head coupling region 142. The plunger head body 134 includes a distal face 135 that in some embodiments may be convex towards the dispensing portion 111. The distal face 135 may have other configurations, as detailed below. The plunger head 150 further comprises a septum 138 extending through the distal face 135 and through which a needle 140 of the plunger (discussed below) extends during a fluid transfer operation. The septum 138 may be positioned at a distal aspect of the plunger head body 134. Optionally, the plunger head 150 may include one or more O-rings 136 positioned between the plunger head body 134 and the inner surface of the reservoir 102. An inner surface of the plunger head 150 and/or plunger head body 134 (e.g., facing towards the plunger 104) may define one or more threads 151 configured to mate with one or more threads 143 on an outer surface of the plunger head coupling region 142. At the completion of a fluid transfer procedure (e.g., when the fluid has been successfully loaded into the reservoir 102, described below), the plunger rod 105 of the plunger 104 can be rotated relative to the plunger head 150 to decouple the plunger rod 105 from the plunger head 150 and enable removal of the plunger rod 105, leaving the plunger head 150 within the reservoir. It will be appreciated that other methods for detachably coupling the plunger rod 105 to the plunger head 150 are possible.

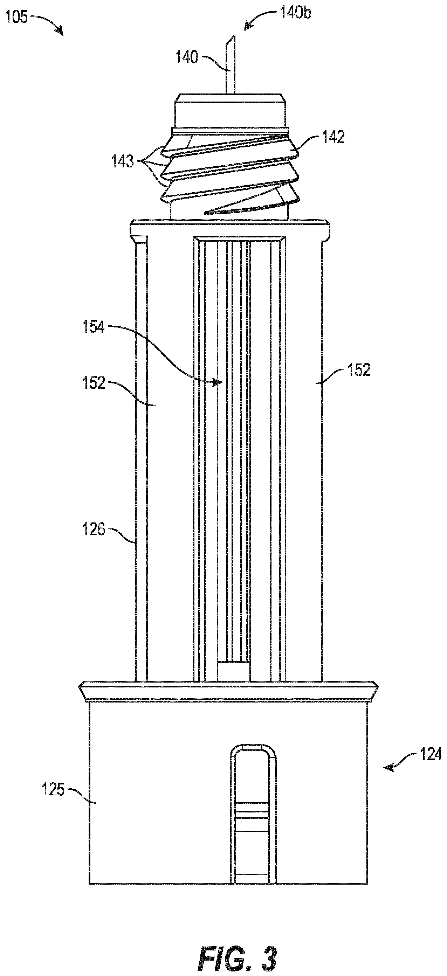

FIG. 3 is an isolated view of the plunger rod 105 of the plunger 104. With reference to FIGS. 1A, 1B, and 3, the shaft 126 of the plunger 104 may comprise a hollow frame formed of a plurality of longitudinally extending struts 152 (FIG. 3) spaced apart circumferentially by windows 154 (FIG. 3). In other examples, the shaft 126 comprises a tubular structure that is circumferentially continuous along its entire length. As shown in FIG. 3, the plunger 104 can include a needle 140 extending longitudinally through the plunger rod 105 and defining a lumen extending therethrough. The needle 140 can have an inlet end 140a (only visible in FIG. 1B) disposed at the vial interface 124 and an outlet end 140b distal of the septum 138 when the plunger rod 105 is coupled to the plunger head 150. The needle 140 can be fixed to a portion of the plunger rod 105 (in this example, at location 160, only visible in FIG. 1B) such that the needle 140 and the plunger rod 105 cannot move axially relative to one another. When the plunger rod 105 is removed from the plunger head 150, the needle 140 withdraws proximally through the septum 138, after which the septum 138 is configured to self-seal.

When an outlet end of a vial is coupled to the vial interface 124 of the plunger 104, the needle 140 extends through a septum of the vial such that the inlet end of the needle 140a is disposed within the vial and in fluid communication with the fluid medication. Proximal movement of the plunger 104 and vial relative to the reservoir 102 draws the fluid medication out of the vial, through the needle 140 and into the interior region 118 of the reservoir 102. As such, the fluid transfer assembly 100 of the present technology is configured to draw a fluid medication into the reservoir 102 through a needle 140 extending into the interior region 118 of the reservoir from the proximal end of the reservoir 102, unlike existing transfer assemblies that place the transfer needle into the interior region through the septum at the distal end portion of the reservoir. Filling from the proximal end of the reservoir 102 also eliminates challenges associated with filling from the distal end, such as leftover fluid medication blocking vents on the connector and distal end of the reservoir.

In some embodiments, in addition to or instead of including one or more filters in the reservoir 102, the transfer assembly 100 may include one or more filters incorporated within the plunger rod 105, in fluid communication with an interior of the needle 140. An example filter 133 is shown schematically in FIG. 1B. Such an arrangement enables filtering of the fluid medication (such as insulin) during filling of the reservoir 102. The filter 133 can be any of the filters disclosed herein.

FIGS. 4A-4D show a method of transferring a fluid medication from a vial V to a reservoir 102 utilizing the fluid transfer assembly 100. As shown in FIG. 4A, the outlet end O of the vial V can be inserted into the cavity 128 at the vial interface 124 of the plunger 104. As the vial V is inserted, the sharp, inlet end 140a (see FIG. 1B) of the needle 140 pierces the septum at the outlet end O of the vial V and passes through the septum into the interior region of the vial V, thereby establishing a fluid connection between the interior region of the fluid vial V and the lumen of the needle 140. In some embodiments, the outlet end O of the vial V can be advanced into the cavity 128 until an end face of the fluid vial abuts the base 131 (FIG. 1B) of the shaft 126, or at least until a bottom ledge of the cap at the outlet end O of the vial V is distal of the annular flange 130 (FIG. 1B) within the cavity 128 (thereby temporarily securing the outlet end O of the vial V within the cavity 128).

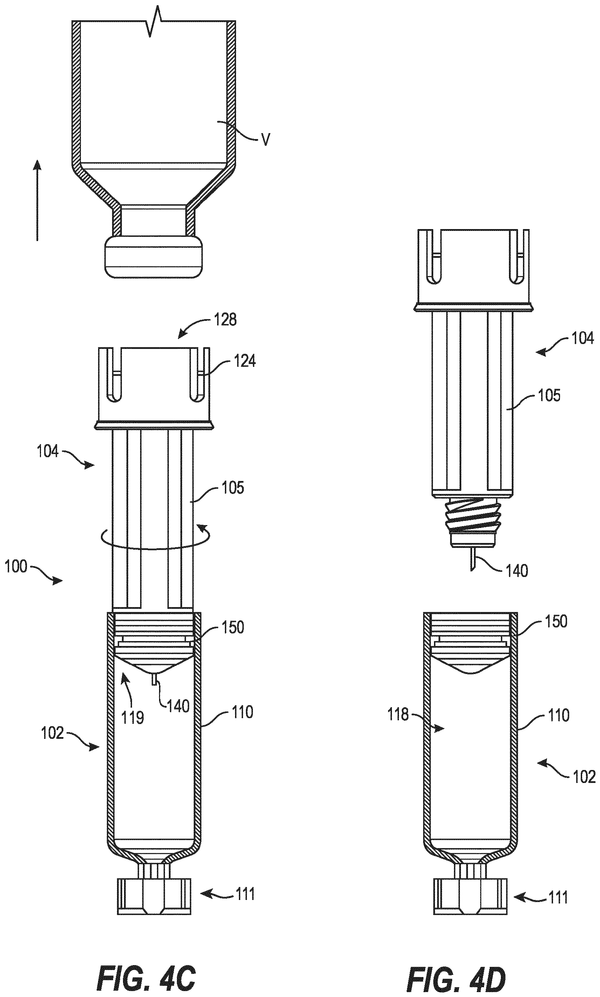

As shown in FIG. 4B, the user can pull the vial V and plunger 104 together proximally, away from the barrel portion 110 of the reservoir 102, thereby creating a negative pressure in the interior region 118 of the barrel portion 110 and forcing the fluid medication within the vial V to flow through the needle 140 and into the interior region 118 of the barrel portion 110. Once the reservoir 102 is filled (or at least filled to the satisfaction of the user), the outlet end of the vial V can be decoupled from the vial interface 124 by withdrawing the vial V proximally from the cavity 128, as depicted in FIG. 4C. The plunger rod 105 can then be decoupled from the plunger head 150, for example by rotating the plunger rod 105 relative to the plunger head 150. Other coupling/decoupling mechanisms are possible. As shown in FIG. 4D, the plunger rod 105 can then be removed from the reservoir 102, leaving only the plunger head 150 within the interior region 118 of the barrel portion 110.

According to some embodiments of the present technology, the plunger head 150 can be configured to address the unique challenges presented by pulling fluid into the reservoir 102 through a needle 140 in the plunger 104 with the plunger 104 in an inverted position (e.g., the plunger 104 is pulled upwardly to fill the reservoir 102). As discussed above, in some embodiments the distal face 135 of the plunger head 150 is convex towards the dispensing portion 111 of the reservoir 102. Because of this, during a filling operation the tip of the needle 140 is positioned below (at a lower elevation than) an annular space 119 (see FIGS. 4B and 4C) between the plunger head 150 and the inner surface of the barrel portion 110. As such, bubbles that may be generated during filling (from air in the vial being pulled into the reservoir 102) travel upwardly and rest within the annular space 119. The bubble(s) may remain trapped within the reservoir such that, when insulin is dispensed from the dispensing end of the reservoir 102 during a dispensing operation, the bubble(s) get expelled along with the insulin, which can affect dosing of the fluid medication among other complications.

To address the foregoing concerns and reduce the presence of bubbles within the reservoir, in some embodiments the plunger head may be shaped such that the needle extending from the distal face of the plunger head is at an elevation higher than the distalmost end of the plunger head during a fluid transfer process, thereby funneling potential bubbles into the needle for removal prior to dispensation of the fluid medication. FIGS. 5A and 5B, for example, show the distal portion of a fluid transfer assembly 500 generally similar to fluid transfer assembly 100, except a plunger 504 of fluid transfer assembly 500 includes a plunger head 550 having an inverted and/or concave distal face 535. As shown, the distal face 535 has an annular region 572 coinciding with the distalmost end of the plunger head 550, and a cavity 574 extending proximally from the annular region 572 into the plunger head body 534. A surface 576 at the distal face 535 defining the cavity 574 may taper proximally such that the cavity 574 forms a funnel. A septum 538 and distal tip of a needle 540 are disposed at the proximal end of the cavity 574. As a result, the distal tip of the needle 540 is positioned at a higher elevation than the distalmost end of the plunger head 550 during a filling procedure. Accordingly, any bubbles that may arise during the filling procedure are funneled upwardly to the needle tip and expelled from the reservoir 502 prior to dispensation of fluid medication from the reservoir 502.

An inner surface 569 at an end portion 566 of a barrel portion 510 may be shaped to mirror that of the plunger distal face 535 to eliminate any dead space between the two. As shown in FIGS. 5A and 5B, in some embodiments the inner surface 569 can extend into an interior region 518, towards the plunger head 550 such that the inner surface 569 is convex towards the plunger head 550.

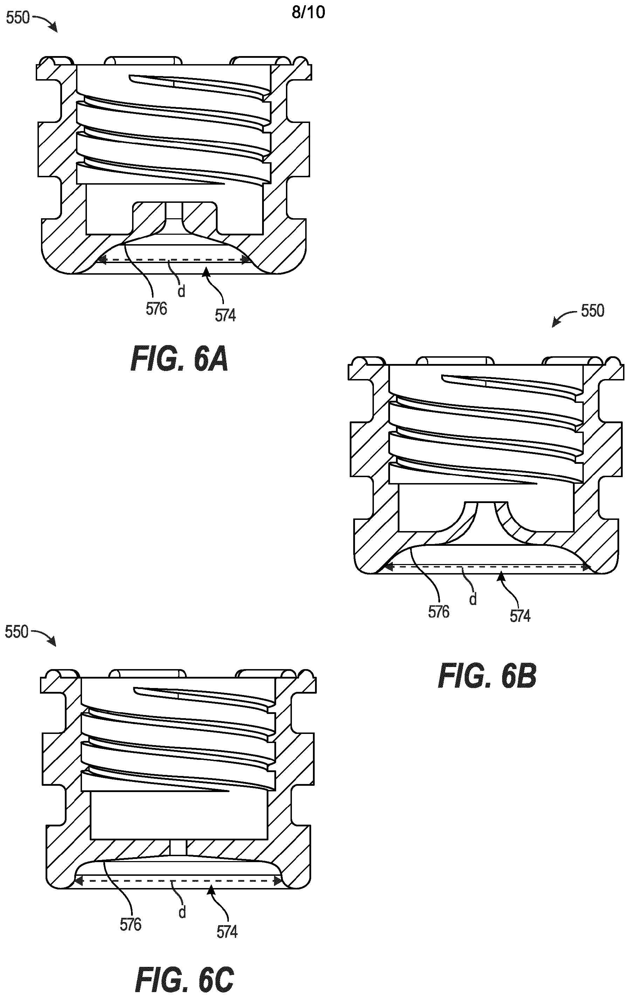

The slope of the surface 576 defining the cavity 574 can be varied. For example, as shown in FIGS. 5A and 5B, the diameter of the cavity 574 may decrease substantially linearly until declining exponentially closer to the septum 538. As shown in FIG. 6A, in some embodiments the diameter d of the cavity 574 decreases exponentially at first, then substantially linearly as the surface 576 approaches the septum (not shown in FIG. 6A). As depicted in FIG. 6B, the diameter d of the cavity 574 may decrease logarithmically. As shown in FIG. 6C, in some embodiments the diameter d of the cavity 574 decreases sharply at first, then substantially linearly and gradually such that a portion of the surface 576 has a nominal slope, thereby creating a shallower cavity 574. Reducing the volume of the cavity 574 can be beneficial as it enables more space within the reservoir 502, so long as the tip of the needle 540 remains proximal of the distalmost end of the plunger head 550. In any of the embodiments disclosed herein, the surface 576 may have one or more inflection points.

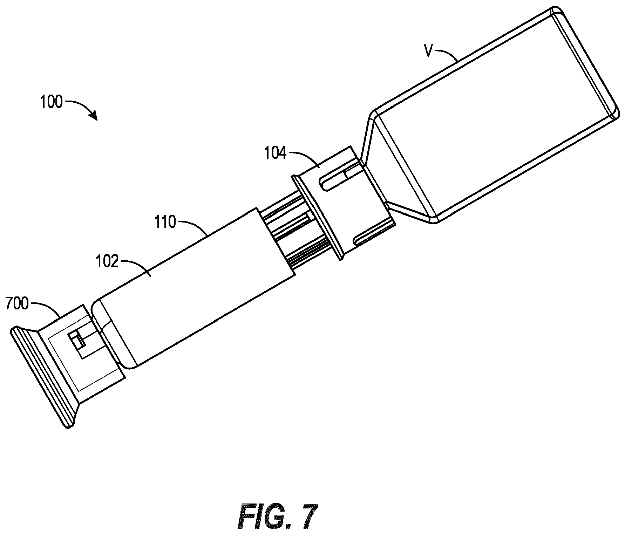

The fluid transfer assemblies of the present technology may optionally include a grip component configured to be detachably coupled to the reservoir and provide a surface for the user to grip when performing a fluid transfer operation. An example grip component 700 is shown coupled to the reservoir 102 of the fluid transfer assembly 100 in FIG. 7. Filling the reservoir 102 by pulling the plunger 104 upwardly, away from the reservoir, requires the user to grasp the reservoir 102, which can be difficult to do without squeezing the barrel portion 110 (which could lead to an incorrect volume of insulin being drawn into the reservoir). The additional grip component alleviates this issue by enabling the patient to grasp the reservoir 102 during a filling operation without touching the barrel portion 110. In some embodiments, for example as shown in FIG. 7, the grip component 700 is configured to be coupled to the dispensing portion 111 (FIG. 1A) of the reservoir 102 by any suitable coupling means (e.g., rotation/a screw arrangement, locking tabs, snap fit, etc.). At least a portion of the grip component 700 can have a greater diameter than that of the barrel portion 110 to facilitate gripping by the user.

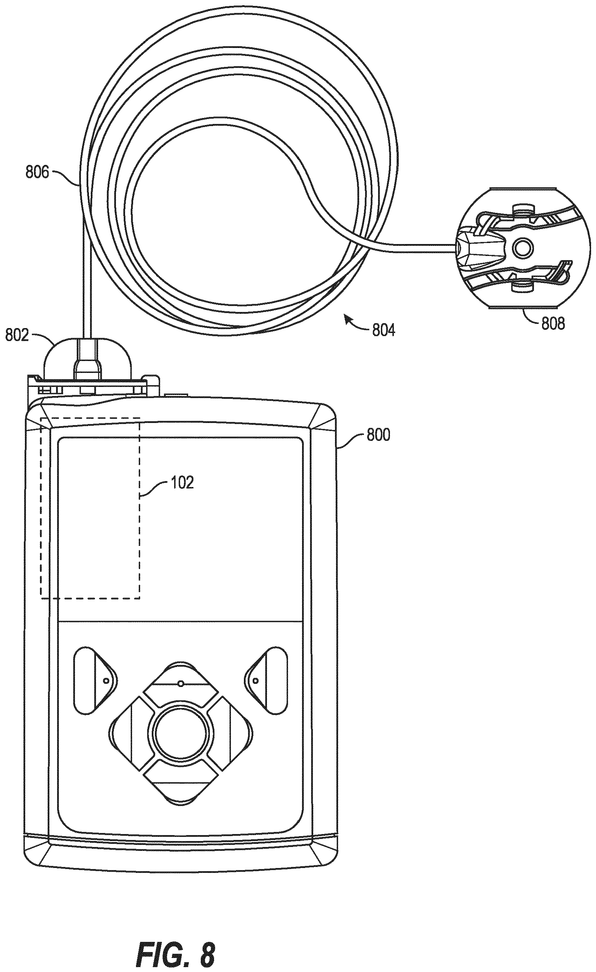

Once filled, the reservoir 102 may then be operatively coupled to an infusion pump. FIG. 8, for example, shows an infusion pump 800 with the reservoir 102 received therein. As depicted, in some examples a connector 802 can be coupled to the dispensing portion 111 (FIG. 1A) of the reservoir 102 to fluidly couple the reservoir 102 to an infusion set 804. An infusion set 804 can comprise, for example, a tubing 806 and an infusion unit 808. The tubing 806 extends between and fluidly couples the connector 802 and the infusion unit 808, and the infusion unit 808 can include a cannula configured for insertion in the patient's skin. In certain embodiments, the connector 802 is coupled to the reservoir 102 prior to coupling the reservoir 102 to the pump 800. In other embodiments, the connector 802 is attached after coupling the reservoir 102 to the pump 800. In another example, the reservoir 102 may be operatively coupled to a patch style infusion device. Such a device adheres to the skin of a user and utilizes a cannula directly inserted into the user without the need for an infusion set. By having the filter or filters reside in the reservoir, various infusion systems may be designed to use a single reservoir design interchangeably, thereby enabling a user to move between, for example, a tethered infusion device and a patch pump without having to have multiple types of reservoirs on hand. Similarly, a user can switch between various infusion sets used with tethered pumps without being limited to infusion sets only incorporating filters. Thus, locating the filters in the reservoir provides better protection for users across various treatment modalities.

### EXAMPLES

Various examples of aspects of the subject technology are described as numbered examples (1, 2, 3, etc.) for convenience. These are provided as examples and do not limit the subject technology.

Example 1: A fluid transfer assembly comprising: a reservoir comprising a sidewall having a proximal end and a distal end and surrounding and defining an interior cavity, the sidewall having an opening at the proximal end, and wherein the reservoir is configured to receive and contain a fluid medication within the interior cavity; a plunger having a proximal end portion and a distal end portion, the plunger comprising: a vial interface at the proximal end portion configured to receive an outlet end of a vial containing a fluid medication; a plunger rod extending distally from the vial interface, wherein the plunger rod is slidably disposed within the interior cavity of the reservoir, and a needle extending longitudinally through the plunger rod, the needle having an inlet end at the vial interface and an outlet end proximate a distal portion of the plunger rod, wherein the needle defines a lumen extending therethrough; wherein the fluid transfer assembly is configured to be coupled to a vial such that, when the outlet end of the vial is coupled to the vial interface of the plunger, the needle extends through a septum of the vial such that the inlet end of the needle is disposed within the vial and in fluid communication with the fluid medication, and wherein, when the needle is fluidly coupled to the vial, proximal movement of the plunger and vial relative to the reservoir draws the fluid medication out of the vial, through the needle, and into the interior region of the reservoir.

Example 2: The fluid transfer assembly of Example 1, wherein the vial interface of the plunger comprises an annular sidewall defining a cavity therein, and wherein the inlet end of the needle is disposed within the cavity, distal of the proximal end of the annular sidewall.

Example 3: The fluid transfer assembly of Example 2, wherein the annular sidewall comprises a flange extending around at least a portion of the circumference of an inner surface of the annular sidewall, wherein the flange projects into the cavity, and wherein the flange is configured to engage the outlet end of the vial to secure the outlet end within the cavity.

Example 4: The fluid transfer assembly of Example 1 or Example 2, wherein: the plunger includes a plunger head coupled to the distal portion of the plunger rod, the plunger head extends between an outer surface of the distal portion of the plunger rod and an inner surface of the sidewall of the reservoir, thereby creating a seal between the plunger and the reservoir.

Example 5: The fluid transfer assembly of Example 4, wherein the plunger head comprises a septum, and wherein the needle extends through the septum such that the outlet end of the needle is distal of a distal end of the septum.

Example 6: The fluid transfer assembly of Example 4, wherein the plunger head extends circumferentially around the distal portion of the plunger rod and includes an inner surface defining a plurality of threads configured to engage a plurality of threads at the outer surface of the distal portion of the plunger rod.

Example 7: The fluid transfer assembly of any one of Examples 1 to 6, wherein the reservoir is configured to be received within a fluid infusion pump.

Example 8: The fluid transfer assembly of any one of Examples 1 to 7, wherein a distal portion of the reservoir includes a filter configured to filter out particulates from the fluid medication when the fluid medication is dispensed from the fluid reservoir.

Example 9: A method for transferring a fluid medication from a vial to a reservoir, the method comprising: providing a fluid transfer assembly, the fluid transfer assembly comprising: a reservoir defining an interior cavity configured to receive and contain a fluid medication; a plunger having a plunger having a proximal end portion and a distal end portion, the plunger comprising: a vial interface at the proximal end portion, a plunger rod extending distally from the vial interface, wherein the plunger rod is slidably positioned within the interior cavity of the reservoir, and a needle extending longitudinally through the plunger, the needle having an inlet end at the vial interface and an outlet end proximate a distal portion of the plunger rod, wherein the needle defines a lumen extending therethrough; coupling an outlet end of a vial containing a fluid medication to the vial interface of the plunger thus piercing a septum of the vial with the inlet end of the needle and establishing a fluid connection between the vial and the lumen of the needle; and while the vial remains engaged to the vial interface of the plunger, moving the vial and plunger together in a proximal direction, away from the reservoir, thereby creating a negative pressure within the interior region of the reservoir and drawing the fluid medication out of the vial, through the needle, and into the interior region of the reservoir.

Example 10: The method of Example 9, wherein coupling the outlet end of the vial to the vial interface comprises inserting the outlet end of the vial distally into a cavity at the proximal end of the plunger.

Example 11: The method of Example 9, further comprising, after drawing a desired amount of fluid medication into the interior region of the reservoir, detaching the vial from the plunger.

Example 12: The method of Example 9, further comprising, after drawing a desired amount of fluid medication into the interior region of the reservoir, removing the plunger from the reservoir.

Example 13: The method of Example 12, wherein: the plunger further comprises a plunger head coupled to the distal portion of the plunger rod and extending between an outer surface of the plunger rod and an inner surface of the reservoir; detaching the plunger rod from the plunger head to enable removal of the plunger from the reservoir, and removing the plunger from the reservoir while the plunger head remains within the reservoir and preventing egress of the fluid medication.

Example 14: The method of Example 13, wherein detaching the plunger rod from the plunger head comprises rotating the plunger rod relative to the plunger head.

Example 15: The method of Example 12, further comprising placing the reservoir in a fluid infusion pump.

Example 16: A reservoir for use with a fluid infusion system for delivering a fluid medication to a user, the reservoir comprising: a barrel portion defining an interior cavity configured to receive a fluid medication; a dispensing portion including a septum, the dispensing portion extending distally from the barrel portion and defining a channel therein, the channel having a proximal end fluidly coupled to the barrel portion and wherein the channel terminates distally at the septum, and wherein, when the fluid medication is dispensed from the reservoir, the fluid medication flows along a flow path starting in the barrel portion and extending through the channel and into a needle extending through the septum; and a filter disposed in the flow path within the channel of the dispensing portion, the filter configured to trap particulates in the fluid medication.

Example 17: The reservoir of Example 16, wherein the dispensing portion comprises a neck extending distally from the barrel portion and a head extending distally away from the neck, wherein the head has a greater cross-sectional dimension than a cross-sectional dimension of the neck, and wherein at least a portion of the filter is positioned within a portion of the channel extending through the neck.

Example 18: The reservoir of Example 17, wherein: the septum is disposed within the head of the dispensing region, the head further includes a receiving cavity comprising a portion of the channel between a distal end of the filter and a proximal end of the septum, and the receiving cavity is configured to receive an inlet end of a needle extending proximally through the septum.

Example 19: The reservoir of Example 16, further comprising a cap configured to be positioned over the dispensing region to secure the septum within the dispensing region.

Example 20: The reservoir of Example 16, wherein the filter is a first filter and the reservoir further comprises a second filter disposed within the dispensing region in the flow path.

Example 21: The reservoir of Example 20, wherein: the dispensing portion comprises a neck extending distally from the barrel portion and a head extending distally away from the neck, wherein the head has a greater cross-sectional dimension than a cross-sectional dimension of the neck, the filter is a first filter and is positioned within a portion of the channel extending through the neck, and the dispensing portion further includes a second filter disposed within the channel, downstream of the first filter.

Example 22: A fluid transfer assembly comprising: a reservoir defining an interior region configured to receive a fluid medication, the reservoir having a proximal end portion and a distal end portion, wherein the reservoir is configured to dispense the fluid medication from the distal end portion; and a plunger having a proximal end portion and a distal end portion, the plunger comprising: a plunger rod configured to be slidably disposed within the interior region of the reservoir, a plunger head configured to be coupled to a distal portion of the plunger rod and disposed within the interior region of the reservoir such that the plunger head provides a seal between the plunger and the reservoir, wherein the plunger head includes a distal face facing a distal inner surface of the reservoir such that a fluid medication contained within the reservoir is bound longitudinally between the distal face of the plunger head and the distal inner surface of the reservoir, and wherein the distal face of the plunger head defines a cavity extending from a distalmost end of the plunger head proximally into the plunger head, wherein, when the reservoir contains a fluid medication, distal movement of the plunger head relative to the reservoir dispenses the fluid medication from the distal end portion of the reservoir.

Example 23: The fluid transfer assembly of Example 22, further comprising a needle extending longitudinally through the plunger rod, the needle having an inlet end at the proximal end portion of the plunger and an outlet end disposed at a proximal portion of the cavity such that the outlet end is proximal of the distalmost end of the plunger head.

Example 24: The fluid transfer assembly of Example 22, wherein a cross-sectional diameter of the cavity tapers proximally.

Example 25: The fluid transfer assembly of Example 22, wherein a shape of the distal inner surface of the reservoir complements a contour of the distal face of the plunger head.

Example 26: The fluid transfer assembly of Example 22, wherein the distal inner surface of the reservoir in convex towards the plunger head.

### Conclusion

It will be appreciated that embodiments in addition to those described herein are within the scope of the technology. Additionally, several other embodiments of the technology can have different configurations, components, or procedures than those described herein. A person of ordinary skill in the art, therefore, will accordingly understand that the technology can have other embodiments with additional elements, or the technology can have other embodiments without several of the features shown and described above with reference to FIGS. 1A-8.

The descriptions of embodiments of the technology are not intended to be exhaustive or to limit the technology to the precise form disclosed above. Where the context permits, singular or plural terms may also include the plural or singular term, respectively. Although specific embodiments of, and examples for, the technology are described above for illustrative purposes, various equivalent modifications are possible within the scope of the technology, as those skilled in the relevant art will recognize. For example, while steps are presented in a given order, alternative embodiments may perform steps in a different order. The various embodiments described herein may also be combined to provide further embodiments.

As used herein, the terms "generally," "substantially," "about," and similar terms are used as terms of approximation and not as terms of degree, and are intended to account for the inherent variations in measured or calculated values that would be recognized by those of ordinary skill in the art.

Moreover, unless the word "or" is expressly limited to mean only a single item exclusive from the other items in reference to a list of two or more items, then the use of "or" in such a list is to be interpreted as including (a) any single item in the list, (b) all of the items in the list, or (c) any combination of the items in the list. Additionally, the term "comprising" is used throughout to mean including at least the recited feature(s) such that any greater number of the same feature and/or additional types of other features are not precluded. It will also be appreciated that specific embodiments have been described herein for purposes of illustration, but that various modifications may be made without deviating from the technology. Further, while advantages associated with certain embodiments of the technology have been described in the context of those embodiments, other embodiments may also exhibit such advantages, and not all embodiments need necessarily exhibit such advantages to fall within the scope of the technology. Accordingly, the disclosure and associated technology can encompass other embodiments not expressly shown or described herein.

Further disclosed herein is the subject-matter of the following clauses:
1. A fluid transfer assembly comprising:
   a reservoir comprising a sidewall having a proximal end and a distal end and surrounding and defining an interior cavity, the sidewall having an opening at the proximal end, and wherein the reservoir is configured to receive and contain a fluid medication within the interior cavity;
   a plunger having a proximal end portion and a distal end portion, the plunger comprising:
      a vial interface at the proximal end portion configured to receive an outlet end of a vial containing a fluid medication;
      a plunger rod extending distally from the vial interface, wherein the plunger rod is slidably disposed within the interior cavity of the reservoir, and
      a needle extending longitudinally through the plunger rod, the needle having an inlet end at the vial interface and an outlet end proximate a distal portion of the plunger rod, wherein the needle defines a lumen extending therethrough;
      wherein the needle has an inlet end at the proximal end portion of the plunger and an outlet end disposed at a proximal portion of the cavity such that the outlet end is proximal of the distalmost end of the plunger head.
      wherein the fluid transfer assembly is configured to be coupled to a vial such that, when the outlet end of the vial is coupled to the vial interface of the plunger, the needle extends through a septum of the vial such that the inlet end of the needle is disposed within the vial and in fluid communication with the fluid medication, and
      wherein, when the needle is fluidly coupled to the vial, proximal movement of the plunger and vial relative to the reservoir draws the fluid medication out of the vial, through the needle, and into the interior region of the reservoir.
2. The fluid transfer assembly of clause 1, wherein the vial interface of the plunger comprises an annular sidewall defining a cavity therein, and wherein the inlet end of the needle is disposed within the cavity, distal of the proximal end of the annular sidewall.
3. The fluid transfer assembly of clause 1 or 2, wherein the annular sidewall comprises a flange extending around at least a portion of the circumference of an inner surface of the annular sidewall, wherein the flange projects into the cavity, and wherein the flange is configured to engage the outlet end of the vial to secure the outlet end within the cavity.
4. The fluid transfer assembly of clause 1 or of any of clauses 1 to 3, wherein:
   the plunger includes a plunger head coupled to the distal portion of the plunger rod,
   the plunger head extends between an outer surface of the distal portion of the plunger rod and an inner surface of the sidewall of the reservoir, thereby creating a seal between the plunger and the reservoir.
5. The fluid transfer assembly of clause 4 or of any of clauses 1 to 4, wherein the plunger head comprises a septum, and wherein the needle extends through the septum such that the outlet end of the needle is distal of a distal end of the septum.
6. The fluid transfer assembly of clause 4 or of any of clauses 1 to 5, wherein the plunger head extends circumferentially around the distal portion of the plunger rod and includes an inner surface defining a plurality of threads configured to engage a plurality of threads at the outer surface of the distal portion of the plunger rod.
7. The fluid transfer assembly of clause 1 or of any of clauses 1 to 6, wherein the reservoir is configured to be received within a fluid infusion pump.
8. The fluid transfer assembly of clause 1 or of any of clauses 1 to 7, wherein a distal portion of the reservoir includes a filter configured to filter out particulates from the fluid medication when the fluid medication is dispensed from the fluid reservoir.
9. The fluid transfer assembly of clause 1 or of any of clauses 1 to 8, wherein a distal inner surface of the reservoir is convex towards the plunger head.
10. A method for transferring a fluid medication from a vial to a reservoir, the method comprising:
   providing a fluid transfer assembly, the fluid transfer assembly comprising:
      a reservoir defining an interior cavity configured to receive and contain a fluid medication;
      a plunger having a proximal end portion and a distal end portion, the plunger comprising:
         a vial interface at the proximal end portion,
         a plunger rod extending distally from the vial interface, wherein the plunger rod is slidably positioned within the interior cavity of the reservoir, and
         a needle extending longitudinally through the plunger, the needle having an inlet end at the vial interface and an outlet end proximate a distal portion of the plunger rod, wherein the needle defines a lumen extending therethrough;
   coupling an outlet end of a vial containing a fluid medication to the vial interface of the plunger thus piercing a septum of the vial with the inlet end of the needle and establishing a fluid connection between the vial and the lumen of the needle; and
      while the vial remains engaged to the vial interface of the plunger, moving the vial and plunger together in a proximal direction, away from the reservoir, thereby creating a negative pressure within the interior region of the reservoir and drawing the fluid medication out of the vial, through the needle, and into the interior region of the reservoir.
11. The method of clause 10, wherein coupling the outlet end of the vial to the vial interface comprises inserting the outlet end of the vial distally into a cavity at the proximal end of the plunger.
12. The method of clause 10 or 11, further comprising, after drawing a desired amount of fluid medication into the interior region of the reservoir, detaching the vial from the plunger.
13. The method of clause 10 or of any of clauses 10 to 12, further comprising, after drawing a desired amount of fluid medication into the interior region of the reservoir, removing the plunger from the reservoir.
14. The method of clause 13 or of any of clauses 10 to 13, wherein:
   the plunger further comprises a plunger head coupled to the distal portion of the plunger rod and extending between an outer surface of the plunger rod and an inner surface of the reservoir,
   detaching the plunger rod from the plunger head to enable removal of the plunger from the reservoir, and
   removing the plunger from the reservoir while the plunger head remains within the reservoir and preventing egress of the fluid medication.
15. The method of clause 14 or of any of clauses 10 to 14, wherein detaching the plunger rod from the plunger head comprises rotating the plunger rod relative to the plunger head.
16. The method of clause 13 or of any of clauses 10 to 15, further comprising placing the reservoir in a fluid infusion pump.
17. A reservoir for use with a fluid infusion system for delivering a fluid medication to a user, the reservoir comprising:
   a barrel portion defining an interior cavity configured to receive a fluid medication;
      a dispensing portion including a septum, the dispensing portion extending distally from the barrel portion and defining a channel therein, the channel having a proximal end fluidly coupled to the barrel portion and wherein the channel terminates distally at the septum, and wherein, when the fluid medication is dispensed from the reservoir, the fluid medication flows along a flow path starting in the barrel portion and extending through the channel and into a needle extending through the septum; and
   a filter disposed in the flow path within the channel of the dispensing portion, the filter configured to trap particulates in the fluid medication.
18. The reservoir of clause 17, wherein the dispensing portion comprises a neck extending distally from the barrel portion and a head extending distally away from the neck, wherein the head has a greater cross-sectional dimension than a cross-sectional dimension of the neck, and wherein at least a portion of the filter is positioned within a portion of the channel extending through the neck.
19. The reservoir of clause 17 or 18, wherein:
   the septum is disposed within the head of the dispensing region,
   the head further includes a receiving cavity comprising a portion of the channel between a distal end of the filter and a proximal end of the septum, and
   the receiving cavity is configured to receive an inlet end of a needle extending proximally through the septum.
20. The reservoir of clause 17 or of any of clauses 17 to 19, further comprising a cap configured to be positioned over the dispensing region to secure the septum within the dispensing region.

## Claims

1. A fluid transfer assembly comprising:
a reservoir comprising a sidewall having a proximal end and a distal end and surrounding and defining an interior cavity, the sidewall having an opening at the proximal end, and wherein the reservoir is configured to receive and contain a fluid medication within the interior cavity;
a plunger having a proximal end portion and a distal end portion, the plunger comprising:
a vial interface at the proximal end portion configured to receive an outlet end of a vial containing a fluid medication;
a plunger rod extending distally from the vial interface, wherein the plunger rod is slidably disposed within the interior cavity of the reservoir, and
a needle extending longitudinally through the plunger rod, the needle having an inlet end at the vial interface and an outlet end proximate a distal portion of the plunger rod, wherein the needle defines a lumen extending therethrough;
wherein the needle has an inlet end at the proximal end portion of the plunger and an outlet end disposed at a proximal portion of the cavity such that the outlet end is proximal of the distalmost end of the plunger head.
wherein the fluid transfer assembly is configured to be coupled to a vial such that, when the outlet end of the vial is coupled to the vial interface of the plunger, the needle extends through a septum of the vial such that the inlet end of the needle is disposed within the vial and in fluid communication with the fluid medication, and
wherein, when the needle is fluidly coupled to the vial, proximal movement of the plunger and vial relative to the reservoir draws the fluid medication out of the vial, through the needle, and into the interior region of the reservoir.

2. The fluid transfer assembly of Claim 1, wherein the vial interface of the plunger comprises an annular sidewall defining a cavity therein, and wherein the inlet end of the needle is disposed within the cavity, distal of the proximal end of the annular sidewall, particularly
wherein the annular sidewall comprises a flange extending around at least a portion of the circumference of an inner surface of the annular sidewall, wherein the flange projects into the cavity, and wherein the flange is configured to engage the outlet end of the vial to secure the outlet end within the cavity.

3. The fluid transfer assembly of Claim 1 or 2, wherein:
the plunger includes a plunger head coupled to the distal portion of the plunger rod,
the plunger head extends between an outer surface of the distal portion of the plunger rod and an inner surface of the sidewall of the reservoir, thereby creating a seal between the plunger and the reservoir.

4. The fluid transfer assembly of one of claims 1 to 3, wherein the plunger head comprises a septum, and wherein the needle extends through the septum such that the outlet end of the needle is distal of a distal end of the septum, and/or wherein the plunger head extends circumferentially around the distal portion of the plunger rod and includes an inner surface defining a plurality of threads configured to engage a plurality of threads at the outer surface of the distal portion of the plunger rod.

5. The fluid transfer assembly of one of claims 1 to 4, wherein the reservoir is configured to be received within a fluid infusion pump, and/or
wherein a distal inner surface of the reservoir is convex towards the plunger head.

6. The fluid transfer assembly of one of claims 1 to 5, wherein a distal portion of the reservoir includes a filter configured to filter out particulates from the fluid medication when the fluid medication is dispensed from the fluid reservoir.

7. A method for transferring a fluid medication from a vial to a reservoir, the method comprising:
providing a fluid transfer assembly, the fluid transfer assembly comprising:
a reservoir defining an interior cavity configured to receive and contain a fluid medication;
a plunger having a proximal end portion and a distal end portion, the plunger comprising:
a vial interface at the proximal end portion,
a plunger rod extending distally from the vial interface, wherein the plunger rod is slidably positioned within the interior cavity of the reservoir, and
a needle extending longitudinally through the plunger, the needle having an inlet end at the vial interface and an outlet end proximate a distal portion of the plunger rod, wherein the needle defines a lumen extending therethrough;
coupling an outlet end of a vial containing a fluid medication to the vial interface of the plunger thus piercing a septum of the vial with the inlet end of the needle and establishing a fluid connection between the vial and the lumen of the needle; and
while the vial remains engaged to the vial interface of the plunger, moving the vial and plunger together in a proximal direction, away from the reservoir, thereby creating a negative pressure within the interior region of the reservoir and drawing the fluid medication out of the vial, through the needle, and into the interior region of the reservoir.

8. The method of Claim 7, wherein coupling the outlet end of the vial to the vial interface comprises inserting the outlet end of the vial distally into a cavity at the proximal end of the plunger.

9. The method of Claim 7 or 8, further comprising, after drawing a desired amount of fluid medication into the interior region of the reservoir, detaching the vial from the plunger.

10. The method of one of claims 7 to 9, further comprising, after drawing a desired amount of fluid medication into the interior region of the reservoir, removing the plunger from the reservoir.

11. The method of one of claims 7 to 10, wherein:
the plunger further comprises a plunger head coupled to the distal portion of the plunger rod and extending between an outer surface of the plunger rod and an inner surface of the reservoir,
detaching the plunger rod from the plunger head to enable removal of the plunger from the reservoir, and
removing the plunger from the reservoir while the plunger head remains within the reservoir and preventing egress of the fluid medication,
particularly
wherein detaching the plunger rod from the plunger head comprises rotating the plunger rod relative to the plunger head.

12. The method of one of claims 7 to 11, further comprising placing the reservoir in a fluid infusion pump.

13. A reservoir for use with a fluid infusion system for delivering a fluid medication to a user, the reservoir comprising:
a barrel portion defining an interior cavity configured to receive a fluid medication;
a dispensing portion including a septum, the dispensing portion extending distally from the barrel portion and defining a channel therein, the channel having a proximal end fluidly coupled to the barrel portion and wherein the channel terminates distally at the septum, and wherein, when the fluid medication is dispensed from the reservoir, the fluid medication flows along a flow path starting in the barrel portion and extending through the channel and into a needle extending through the septum; and
a filter disposed in the flow path within the channel of the dispensing portion, the filter configured to trap particulates in the fluid medication.

14. The reservoir of Claim 13, wherein the dispensing portion comprises a neck extending distally from the barrel portion and a head extending distally away from the neck, wherein the head has a greater cross-sectional dimension than a cross-sectional dimension of the neck, and wherein at least a portion of the filter is positioned within a portion of the channel extending through the neck,
particularly wherein:
the septum is disposed within the head of the dispensing region,
the head further includes a receiving cavity comprising a portion of the channel between a distal end of the filter and a proximal end of the septum, and
the receiving cavity is configured to receive an inlet end of a needle extending proximally through the septum.

15. The reservoir of Claim 13 or 14, further comprising a cap configured to be positioned over the dispensing region to secure the septum within the dispensing region.
